# EUROPEAN PATENT APPLICATION

(11) **EP 4 063 270 A1**
(43) Date of publication of application: **28.09.2022**
(21) Application number: 22163831.5
(22) Date of filing: 23.03.2022
(51) Int. Cl.: B64D 11/02, A61L 2/10, B64F 5/30

(54) **INTELLIGENT, CONFIGURABLE, AND CONNECTED OPERATION OF UV LAMPS IN AIRCRAFT LAVATORY**

(30) Priority: 23.03.2021 US 202117209684
(71) Applicant: B/E Aerospace, Inc., Winston-Salem, NC 27105 (US)
(72) Inventor: EDQUIST, John, Milwaukee, 53207 (US); JOHANNESSEN, Eric, Holbrook, 11741 (US); BAJEKAL, Sanjay, Simsbury, CT, 06070 (US)
(74) Representative: Dehns

(57) **Abstract**

A system for centrally controlled sanitization of a lavatory of an aircraft using ultraviolet (UV) light includes a first UV light source (502) configured to emit a first UV light (504), and a second UV light source (510) configured to emit a second UV light (512). The system further includes a central controller (102) coupled to the first UV light source and the second UV light source and configured to independently control the first UV light source and the second UV light source to emit the first UV light and the second UV light at least one of at different times or for different durations.

## Description

### FIELD

The present disclosure relates to systems and methods for centralized intelligent control of ultraviolet light sources within an aircraft lavatory by a central controller.

### BACKGROUND

Ultraviolet (UV) lighting technologies perform germicidal functions but may also present impacts to human health and may degrade materials exposed to the UV light. Furthermore, lamps which emit UV light may have relatively short lifetimes, and these lamps draw a relatively large amount of power. Due to these limitations, conventional UV lighting architecture is expensive to implement in aircraft and results in significant downtime as a result of cessation of operation of UV lamps.

It is therefore desirable to develop systems and methods that allow UV light sources to be implemented in aircraft that overcome the shortfalls of conventional technology.

### SUMMARY

Disclosed herein is a system for centrally controlled sanitization of a lavatory of an aircraft using ultraviolet (UV) light. The system includes a first UV light source configured to emit a first UV light, and a second UV light source configured to emit a second UV light. The system further includes a central controller coupled to the first UV light source and the second UV light source and configured to independently control the first UV light source and the second UV light source to emit the first UV light and the second UV light at least one of at different times or for different durations.

Any of the foregoing embodiments may further include at least one sensor configured to detect data corresponding to usage of the lavatory, wherein the central controller is further configured to control at least one of the first UV light source or the second UV light source based on the data corresponding to the usage of the lavatory.

In any of the foregoing embodiments, the data corresponding to the usage of the lavatory indicates a used portion of the lavatory, wherein the central controller is further configured to control the at least one of the first UV light source or the second UV light source to illuminate the used portion of the lavatory with the UV light.

In any of the foregoing embodiments, the at least one sensor includes at least one of: a touchless seat sensor configured to receive a toilet seat request corresponding to a request to lift a toilet seat of the lavatory; a touchless soap sensor configured to receive a soap dispense request corresponding to a request to dispense soap within the lavatory; a touchless faucet sensor configured to detect a faucet request corresponding to a request to operate a faucet of the lavatory; a touchless flush sensor configured to detect a flush request corresponding to a request to flush a toilet of the lavatory; or a touchless trash sensor configured to detect a trash flap request corresponding to a request to actuate a trash flap of the lavatory.

In any of the foregoing embodiments, the central controller is further configured to determine a power threshold corresponding to a maximum amount of power available to the system, and to control the first UV light source and the second UV light source to cause a total power draw of the system to remain at or below the power threshold.

Any of the foregoing embodiments may further include a memory configured to store parameters corresponding to the first UV light source and the second UV light source, wherein: the parameters include at least one of a turn-on time, a power of the first UV light or the second UV light, or a peak wavelength of the first UV light or the second UV light; and the central controller is further configured to control the first UV light source and the second UV light source based on the parameters.

In any of the foregoing embodiments, the parameters are adjustable by at least one of an operator of the system or the central controller.

In any of the foregoing embodiments, the central controller is further configured to determine a health status of the system including at least one of a remaining life of the first UV light source, a remaining life of the second UV light source, or surface wear experienced by a surface of the lavatory in response to the first UV light or the second UV light.

In any of the foregoing embodiments, the first UV light and the second UV light each have a peak wavelength that is 200 between nanometers and 230 nanometers (0.00787 thousandths of an inch and 0.00906 thousandths of an inch).

Also disclosed is a system for centrally controlled sanitization of a lavatory of an aircraft using ultraviolet (UV) light. The system includes a first UV light source configured to emit a first UV light, and a second UV light source configured to emit a second UV light. The system further includes at least one sensor configured to detect data corresponding to usage of the lavatory. The system further includes a central controller coupled to the first UV light source, the second UV light source, and the at least one sensor, and configured to independently control the first UV light source and the second UV light source based on the data corresponding to the usage of the lavatory.

In any of the foregoing embodiments, the data corresponding to the usage of the lavatory indicates a used portion of the lavatory, wherein the central controller is further configured to control the at least one of the first UV light source or the second UV light source to illuminate the used portion of the lavatory with the UV light.

In any of the foregoing embodiments, the at least one sensor includes at least one of: a touchless seat sensor configured to receive a toilet seat request corresponding to a request to lift a toilet seat of the lavatory; a touchless soap sensor configured to receive a soap dispense request corresponding to a request to dispense soap within the lavatory; a touchless faucet sensor configured to detect a faucet request corresponding to a request to operate a faucet of the lavatory; a touchless flush sensor configured to detect a flush request corresponding to a request to flush a toilet of the lavatory; or a touchless trash sensor configured to detect a trash flap request corresponding to a request to actuate a trash flap of the lavatory.

In any of the foregoing embodiments, the central controller is further configured to determine a power threshold corresponding to a maximum amount of power available to the system, and to control the first UV light source and the second UV light source to cause a total power draw of the system to remain at or below the power threshold.

Any of the foregoing embodiments may further include a memory configured to store parameters corresponding to the first UV light source and the second UV light source, wherein: the parameters include at least one of a turn-on time, a power of the first UV light or the second UV light, or a peak wavelength of the first UV light or the second UV light; and the central controller is further configured to control the first UV light source and the second UV light source based on the parameters.

Also disclosed is a method for centrally controlled sanitization of a lavatory of an aircraft using ultraviolet (UV) light. The method includes providing, in the lavatory, a first UV light source configured to emit a first UV light and a second UV light source configured to emit a second UV light. The method further includes independently controlling, by a central controller, the first UV light source and the second UV light source to emit the first UV light and the second UV light at least one of at different times or for different durations.

Any of the foregoing embodiments may further include detecting, by a sensor, data corresponding to usage of the lavatory, wherein independently controlling the first UV light source and the second UV light source includes controlling at least one of the first UV light source or the second UV light source based on the data corresponding to the usage of the lavatory.

In any of the foregoing embodiments, the data corresponding to the usage of the lavatory indicates a used portion of the lavatory, and wherein independently controlling the first UV light source and the second UV light source includes controlling the at least one of the first UV light source or the second UV light source to illuminate the used portion of the lavatory with the UV light.

Any of the foregoing embodiments may further include determining, by the central controller, a power threshold corresponding to a maximum amount of power available to the lavatory; and causing, by the central controller, a total power draw of components of the lavatory to remain at or below the power threshold.

Any of the foregoing embodiments may further include storing, in a memory, parameters corresponding to the first UV light source and the second UV light source, wherein: the parameters include at least one of a turn-on time, a power of the first UV light or the second UV light, or a peak wavelength of the first UV light or the second UV light; and independently controlling the first UV light source and the second UV light source is based on the parameters.

Any of the foregoing embodiments may further include determining, by the central controller, a health status of components of the lavatory including at least one of a remaining life of the first UV light source, a remaining life of the second UV light source, or surface wear experienced by a surface of the lavatory in response to the first UV light or the second UV light.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the detailed description and claims when considered in connection with the figures, wherein like numerals denote like elements.
FIG. 1 illustrates a system for central control of sanitization of an aircraft lavatory using UV light, in accordance with various embodiments;
FIG. 2 illustrates additional details of the system of FIG. 1, in accordance with various embodiments;
FIG. 3 illustrates additional details of the system of FIG. 1, in accordance with various embodiments;
FIG. 4 illustrates a method for central control of sanitization of an aircraft lavatory using UV light, in accordance with various embodiments;
FIGS. 5A, 5B, 5C, 5D, and 5E illustrate a system for centralized control of sanitization of an aircraft lavatory by multiple UV light sources, in accordance with various embodiments;
FIG. 6 illustrates a system for controlling various lavatories of an aircraft that each include systems for central control of sanitization thereof, in accordance with various embodiments; and
FIG. 7 illustrates an exemplary implementation of the method of FIG. 4 by the system of FIG. 1, in accordance with various embodiments.

### DETAILED DESCRIPTION

The detailed description of exemplary embodiments herein makes reference to the accompanying drawings, which show exemplary embodiments by way of illustration. While these exemplary embodiments are described in sufficient detail to enable those skilled in the art to practice the exemplary embodiments of the disclosure, it should be understood that other embodiments may be realized and that logical changes and adaptations in design and construction may be made in accordance with this disclosure and the teachings herein. Thus, the detailed description herein is presented for purposes of illustration only and not limitation. The steps recited in any of the method or process descriptions may be executed in any order and are not necessarily limited to the order presented.

Furthermore, any reference to singular includes plural embodiments, and any reference to more than one component or step may include a singular embodiment or step. Also, any reference to attached, fixed, connected or the like may include permanent, removable, temporary, partial, full and/or any other possible attachment option. Additionally, any reference to without contact (or similar phrases) may also include reduced contact or minimal contact. Surface shading lines may be used throughout the figures to denote different parts but not necessarily to denote the same or different materials.

The present disclosure is directed to a centralized intelligent controller for controlling various features of an aircraft lavatory. The systems and methods herein provide a connected system of ultraviolet (UV) light sources that are each targeted towards specific surfaces and are each controlled by a central controller. Operation of each lamp is controlled individually by the central computer and is based on several inputs such as lavatory occupancy, use detection of various portions of the lavatory, and power available to the lavatory system. The operation of each UV light source is configurable based on operator specifications such as a desired percent of disinfection, a target pathogen to be injured, neutralized, killed, or the like.

The ultraviolet light sources of the present disclosure may emit far UV-C light. This light may be defined as a germicidal light source having a peak wavelength that is between 200 nanometers (200 nm, 0.00787 thousandths of an inch, or mils) and 230 nm (0.00906 mils), between 210 nm (0.00827 mils) and 225 nm (0.00886 mils), or about 222 nm (0.00874 mils). Where used in this context, "about" refers to the referenced value plus or minus 10 percent of the referenced value.

UV light of this type may effectively injure, neutralize, or kill pathogens that are both airborne and resting on surfaces. In addition, this light may be readily absorbed by most materials and may be relatively safe for human exposure. However, UV-C light may cause discoloration or other degradation of certain materials over time. An exemplary UV-C light source may have a lifetime of thousands of hours.

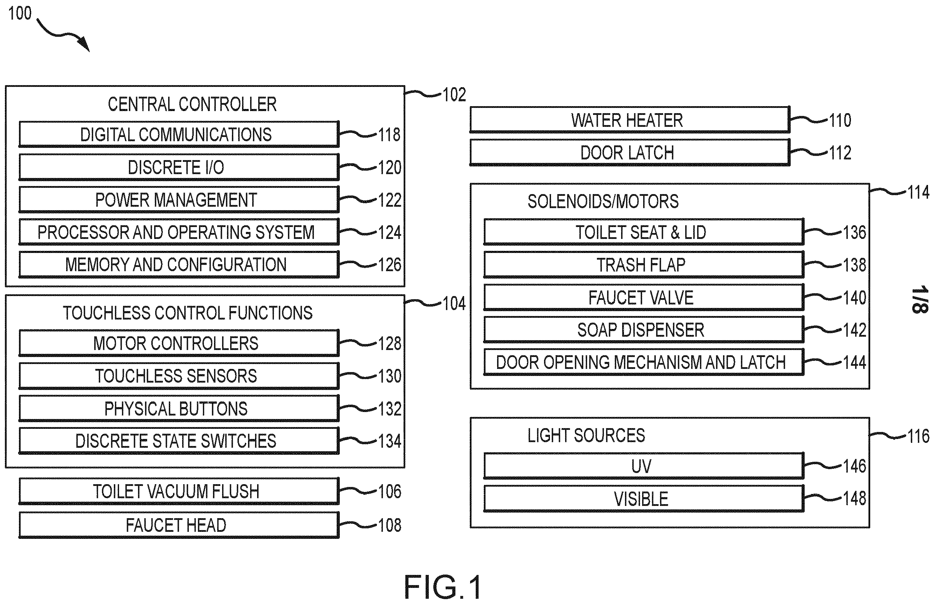

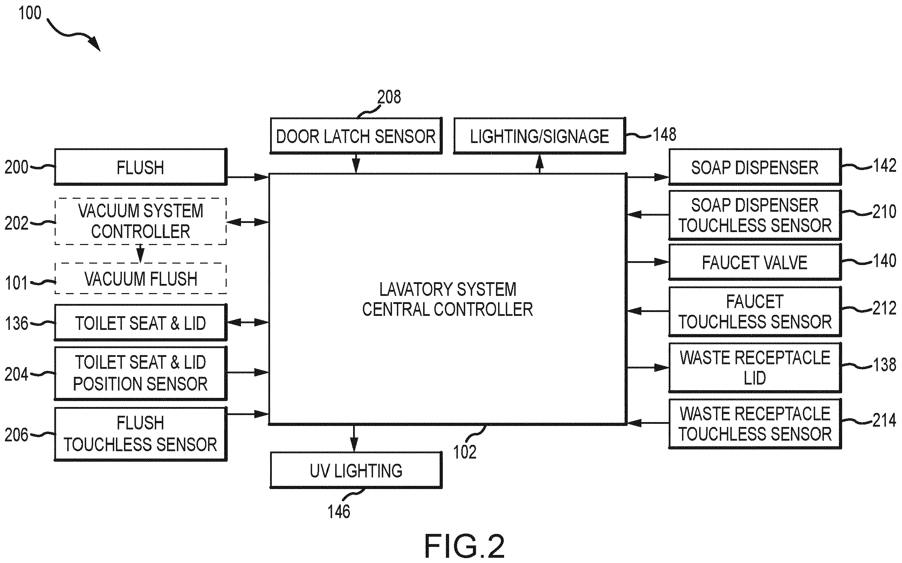

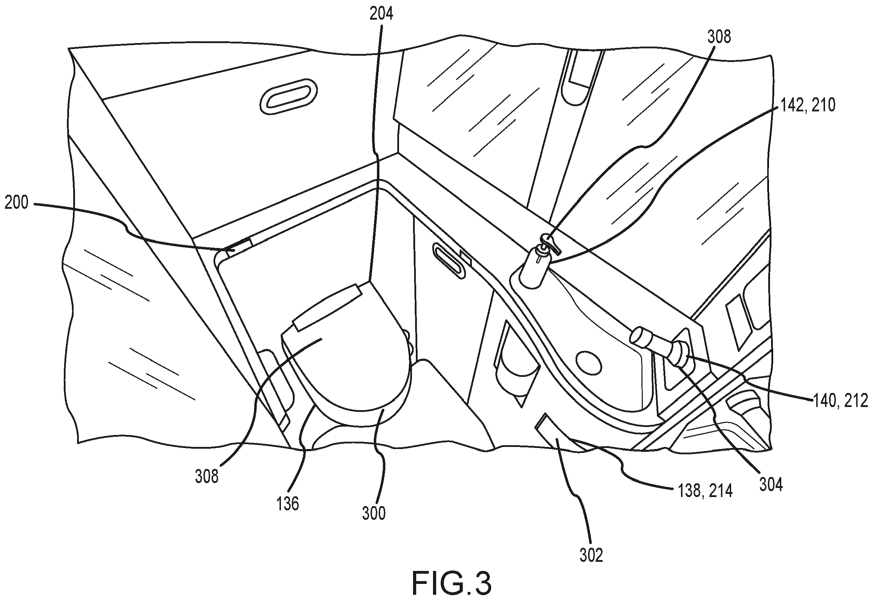

Referring generally to FIGS. 1, 2, and 3, an exemplary system 100 for centrally controlled sanitization of an aircraft lavatory includes multiple sensors, multiple actuators (which may include additional features such as a vacuum flush motor, a water heater, or the like), multiple light sources, and a central controller 102. The system 100 may further include touchless control functions 104, a toilet vacuum flush 106, a faucet head 108, a water heater 110, a door latch 112, multiple solenoids or motors 114, and multiple light sources 116.

The central controller 102 may be coupled to each feature of the system 100 and may include various units or modules capable of performing various functions. In particular, the central controller 102 may include a digital communications function 118 capable of communicating with various features of the system 100. The central controller 102 may further have an input/output function 120 capable of managing inputs and outputs of the central controller 102. The central controller 102 may further include a power management function 122 capable of monitoring and controlling power management of the system 100. The central controller 102 may further include one or more processor or controller and a corresponding operating system 124. The processor or controller may include a logic device such as one or more of a central processing unit (CPU), an accelerated processing unit (APU), a digital signal processor (DSP), a field programmable gate array (FPGA), an application specific integrated circuit (ASIC), or any other device capable of implementing logic. In various embodiments, the processor or controller may further include any non-transitory memory known in the art. The memory may store instructions usable by the logic device to perform operations as described herein. The central controller 102 may also include a memory 126 which may store airline-specific preferences and operations (such as a desirable lighting configuration or desirable ultraviolet emission time) or other information.

The touchless control functions 104 may include various features (e.g., input devices such as motion sensors or buttons) to facilitate touchless or other control of functions of the system 100. For example, the central controller 102 may control various operations within the lavatory based on information received from the touchless control functions 104. In particular, the touchless control functions may include motor controllers 128 that are each coupled to one or more solenoid, motor, or actuator of the system 100. For example, each solenoid, motor, or actuator may include a specific motor controller 128 which controls operation of the specific solenoid, motor, or actuator based on signals received from the central controller 102. The touchless control functions 104 may further include a plurality of touchless sensors 130. The touchless sensors 130 may include sensors capable of receiving user input such as a proximity sensor, a motion sensor, or the like. The user input detected by the touchless sensors 130 may correspond to a request for operation of one or more component of the system 100 (e.g., a toilet flush request). The touchless control functions 104 may further include physical buttons 132 which are likewise capable of receiving user input corresponding to a request for operation of one or more component of the system 100. The touchless control functions 104 may also include discrete state switches 134. The discrete state switches 134 may include any type of discrete switch such as a limit switch (which turn on or off in response to contact with a part or element), a reed switch (featuring a mechanical switch dependent on a position of a magnet), a proximity switch (which operate on the principles of induction), photoelectric sensors (for example, usable in part detection or material handling), or the like. The discrete state switches 134 may cause various motors or actuators to remain in a desired state.

The toilet vacuum flush 106 may include an actuator, motor, or other device or component which initiates a vacuum flush of a toilet within the laboratory. The toilet vacuum flush 106 may be controlled to initiate or cease a flush operation based on instructions received or determined by the central controller 102.

The faucet head 108 may include a sink faucet through which water may flow. A valve (faucet valve, or sink valve, 140) may be located upstream from the sink faucet and may be controlled by the central controller 102 to adjust an amount of water flowing through the faucet head 108.

The water heater 110 may include a tank or tankless water heater capable of warming water that is used in the lavatory (e.g., water flowing through the faucet head 108). The central controller 102 may control operation of the water heater 110 based on various elements of information such as information received from the touchless control functions 104, a power status of the system 100, or the like.

The door latch 112 may be used to lock or unlock a door leading into the lavatory. The door latch 112 may be automatically controlled by the central controller 102, may be manually controlled by a user (e.g., by flipping a manual switch), or the like. The door latch 112 may include a sensor that transmits information to the central controller 102 indicating whether the door latch 112 is in a locked or unlocked state.

The solenoids or motors 114 (which may also be referred to as actuators) may include various solenoids or motors capable of actuating or controlling operation of functions of the system 100. In addition, the touchless control functions 104 may include touchless or touch-activated sensors that detect a request to actuate the motor or solenoid 114 or detect other data corresponding to the system 100. In particular, the solenoids or motors 114 may include one or more toilet seat and lid motor, or actuator, 136. The toilet seat and lid motor 136 may control each of a toilet seat and a toilet lid 300 to actuate between a raised position and a lowered position. In particular the motor 136 may control the toilet seat and lid 300 to both be raised, both be lowered, or the lid raised and the seat lowered. The system 100 may further include a toilet seat and lid position sensor 204 that detects data corresponding to a state of the toilet seat and lid 300 (i.e., raised, lowered, or raised and lowered). In various embodiments, the toilet seat and lid position sensor 204 may also or instead include a touchless seat sensor capable of receiving user input corresponding to a request to lower or raise at least one of the toilet seat or the toilet lid.

The solenoids and motors 114 may also include a trash flap motor, or actuator, 138. The trash flap motor 138 may control a trash flap 302 to alternate between an open position and a closed position. The system 100 may further include a touchless trash sensor, or waste receptacle touchless sensor, 214. The trash sensor 214 may detect a request for the trash flap 302 to be opened. For example, a user may waive a hand in front of the trash sensor 214 to cause the trash flap 302 to open.

The solenoids and motors 114 may further include a faucet valve 140, or faucet actuator, 140 coupled to a faucet 304. The faucet valve 140 may open to allow water to flow through the faucet 304 and may close to reduce the likelihood of water flowing through the faucet 304. The system 100 may further include a touchless faucet sensor 212 capable of receiving user input corresponding to a request to at least one of open or close the faucet valve 140.

The solenoids and motors 114 may also include a soap dispenser, or soap actuator, 142. The soap dispenser 142 may be capable of pumping soap through an outlet or nozzle 306. The system 100 may also include a touchless soap sensor 210 capable of receiving user input corresponding to a request for soap to be dispensed from the outlet or nozzle 306.

The solenoids and motors 114 may further include a door opening mechanism and latch 144. The door opening mechanism and latch 144 may include a motor, actuator, or the like capable of opening a door to the lavatory and closing the door to the lavatory. The door opening mechanism and latch 144 may further include a latch capable of locking the lavatory door in a locked position and unlocking the lavatory door. The central controller 102 may receive information from the door opening mechanism and latch 144 indicating at least one of a current state or a previous state of the door opening mechanism and latch 144.

The system 100 may further include a flush button 200 capable of receiving user input corresponding to a request to flush a toilet 308 (e.g., via depression of the flush button 200). The system 100 may also include a touchless flush sensor 206 capable of receiving user input corresponding to a request to flush the toilet 308 (e.g., by waving a hand in front of the touchless flush sensor 206). The system 100 may further include a vacuum system controller 202 coupled to the vacuum flush 106. The vacuum system controller 202 may control the vacuum flush 106 to flush the toilet 308 based on user input received by at least one of the flush button 200 or the touchless flush sensor 206.

The light sources 116 may include one or more UV light source 146 and one or more visible light source 148. The UV light source 146 may include a light source that generates ultraviolet light having a wavelength that injures, neutralizes, or kills pathogens, such as a peak wavelength of 222 nm. The UV light source 146 may include multiple UV light sources that are each directed to a separate portion of the lavatory as will be discussed below. For example, a first UV light source may be directed towards the toilet 308 and a second UV light source may be directed towards the faucet 304.

The visible light source 148 may include a first one or more visible light source that directs light into the lavatory to illuminate the lavatory for ease of use of a user. The visible light source 148 may further include signs indicating a current status of the lavatory (e.g., occupied or vacant).

The central controller 102 may receive information from each component of the system 100 (i.e., a status of one or more of the solenoids and motors 114, user input or other information detected or received by the touchless control functions 104, a status of one or more of the light sources 116 or other system components, or the like). The central controller 102 is further capable of making an inference (e.g., an area of the lavatory that was used) based on the detected or received data, and of controlling various elements of the system 100 (such as multiple UV light sources) based on the inference. The central controller 102 is further capable of managing power drawn by each component of the system 100 and controlling the components to cause a total power draw to be less than or equal to a threshold power level that corresponds to a maximum amount of power that is available to the system 100. The central controller 102 is also capable of monitoring a health status of various components of the system (i.e., remaining lives or an amount of wear and tear experienced by surfaces as a result of the UV light sources 146) or the like, and to take an action based on the health status.

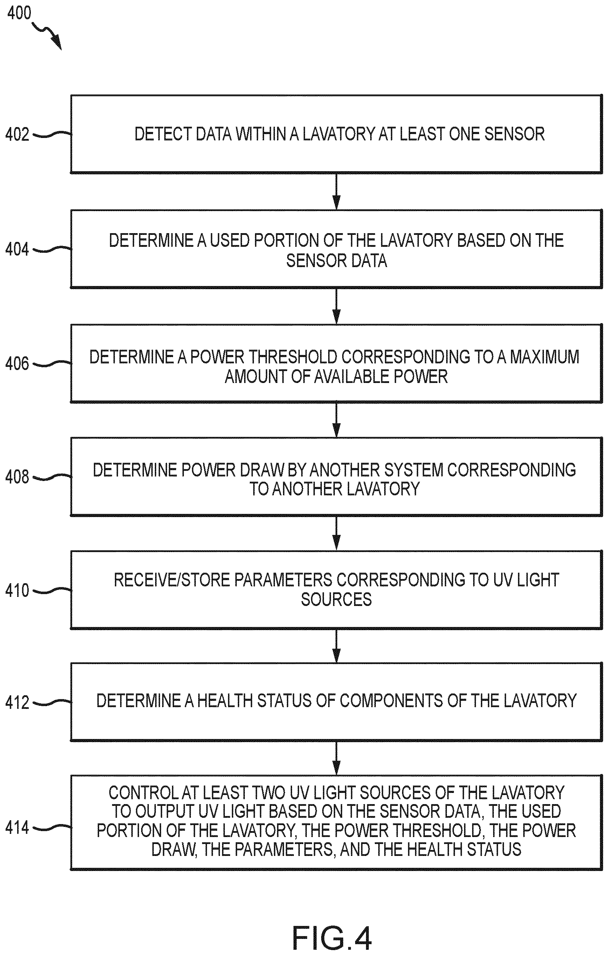

Referring now to FIG. 4, a method 400 for centralized controlled sanitization of an aircraft lavatory using UV light is shown. In block 402, one or more sensor of the lavatory may detect data corresponding to the lavatory. For example, the sensor may determine whether the lavatory is occupied, a current or previous status of one or more component of the lavatory (such as a toilet seat, a door, or a latch), whether one or more light source is generating light, a quantity of remaining supplies, an area of the lavatory that was used by a previous user, or the like. The sensor may also detect user input corresponding to a request for operation of one or more feature of the lavatory such as a flush request, a request to dispense soap or water, a request to raise or lower a toilet seat or toilet lid, a request to open or close a trash flap, or the like. The detected data may be transmitted to a central controller of the system.

In block 404, the central controller may determine a used portion of the lavatory based on the sensor data. For example, the central controller may infer a portion of the lavatory that was used by a previous user. The central controller may infer, for example, that the previous user used the toilet if the toilet seat was raised, may infer that the previous user touched the trash can if the trash can lid was moved, may infer that the sink was used if the previous user requested soap to be dispensed, or the like.

In block 406, the central controller may determine a power threshold that corresponds to a maximum amount of power that is available to the lavatory. The central controller may determine the power threshold by accessing a memory (i.e., the memory may be programmed with the power threshold), may receive the power threshold from an operator of the aircraft, may calculate the power threshold based on a received power signal, or the like.

In block 408, the central controller may determine a current amount of power drawn by another system that corresponds to another lavatory on the aircraft. For example, the central controller may make this determination based on information received from various components of the other lavatory or from a signal received by a central controller that is responsible for the other lavatory. In various embodiments, the central controller may also determine, or access in a memory, a prioritization of each component of the multiple lavatories. The priorities may be programmed into the system by an operator of the aircraft and may prioritize various functions within the lavatory such as UV disinfecting, visible lighting, toilet flushes, or the like.

In block 410, the central controller may at least one of receive or store parameters corresponding to multiple UV light sources within the lavatory. For example, these parameters may include a turn-on time corresponding to a desired amount of time for one or more UV light source to remain on to achieve a desired amount of disinfection (e.g., 15 seconds may correspond to 90 percent of disinfection and 30 seconds may correspond to 99.9 percent of disinfection). As another example, the parameters may include desirable power output of at least one UV light source (a higher power output may correspond to greater disinfection). As another example, the parameters may include a desirable peak wavelength of at least one UV light source. These parameters may be set by an aircraft operator based on desired goals (such as a percent of disinfection desired, a specific pathogen that is likely to be carried by one or more passenger and an optimal wavelength of light to injure the pathogen, or the like). In that regard, the aircraft operator may adjust or change the stored parameters based on changing situations and locations in the world.

In block 412, the central controller may determine a health status of one or more component of the lavatory. The central controller may, for example, determine the health status based on the data that was detected in block 402, based on usage information of each UV light source, or the like. For example, the central controller may record a duration of each use of an ultraviolet light and a duration for which one or more surface was exposed to the ultraviolet light. The central controller may be programmed to know that certain surfaces degrade to or past a threshold degradation level in response to a first duration of exposure of ultraviolet light. The central controller may determine the health status of the various surfaces based on the duration of each use, the material of one or more exposed surfaces, and the threshold duration. The central controller may, for example, determine that a bulb within a UV light source is nearing the end of its expected lifetime based on a cumulative duration of use of the bulb. For example, if the expected lifetime is 1,000 hours of cumulative use, the central controller may determine that the bulb is nearing the end of its expected life in response to the cumulative use of the bulb reaching 900 hours.

In block 414, the central controller may control at least two UV light sources individually based on the sensor data, the determined used portion of the lavatory, the power threshold, the power draw by the other system, the parameters, and the health status. An exemplary use of the sensor data is that the central controller may only control a UV light source to illuminate within the lavatory in response to the lavatory being vacant in order to reduce exposure of humans to the UV light. An exemplary use of the used portion of the lavatory is that the central controller may only cause UV light sources to illuminate portions of the lavatory that were used in a previous use. An exemplary use of the power threshold is that the central controller may only control a UV light source to emit light if the total power draw with the UV light source emitting the light is less than or equal to the power threshold. An exemplary use of the power draw by other lavatories is that the central controller may only control a UV light source to emit light if the total power draw by all lavatory systems of the aircraft is less than or equal to a total power threshold corresponding to a maximum amount of power draw for a combination of each lavatory. An exemplary use of the parameters is that the central controller may control each light source to emit the light for a duration equal to the turn-on time. An exemplary use of the health status is that the central controller may control a light source nearing its expected end of life to emit light for a duration that is less than a stored turn-on time.

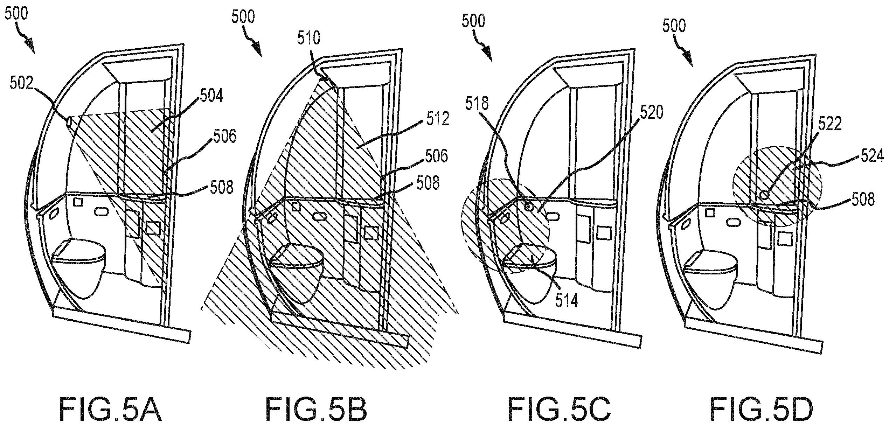

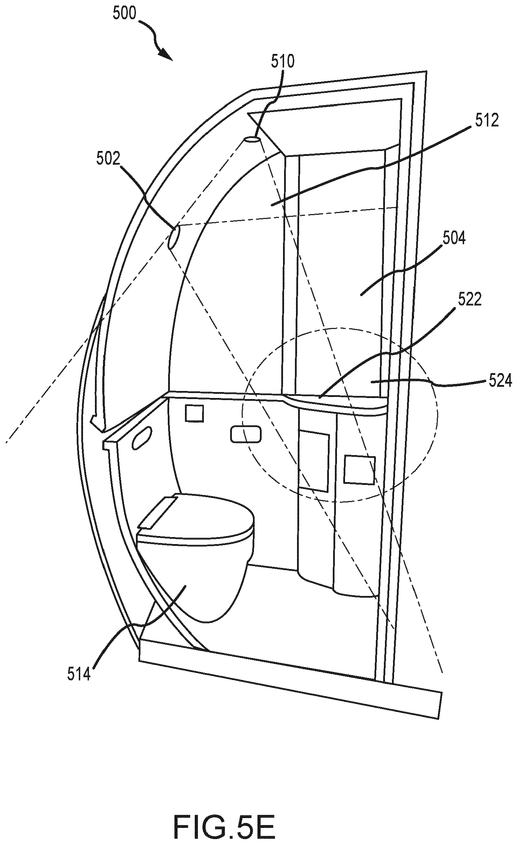

Turning to FIGS. 5A, 5B, 5C, 5D, and 5E, an exemplary system 500 for centrally controlled sanitization of a lavatory of an aircraft using UV light is shown. The system 500 includes multiple UV light sources that each emit light towards, and illuminate for the purpose of disinfecting, a portion of the lavatory. A first UV light source 502 emits a first UV light 504 that illuminates, and disinfects, a door 506 and a sink 508 of the lavatory. A second UV light source 510 emits a second UV light 512 that illuminates, and disinfects, the door 506, the sink 508, a toilet 514, and a floor 516. A third UV light source 518 emits a third UV light 520 that illuminates, and disinfects, the toilet 514. The third UV light 520 may further illuminate a seat cover and seat lid of the toilet 514. A fourth UV light source 522 emits a fourth UV light 524 that illuminates, and disinfects, the sink 508 (and potentially other surfaces such as a trash can lid, a toilet paper holder, or the like).

An intensity of each UV light that reaches each surface may be different. For example, an intensity of the second UV light 512 that reaches the door 506 may be different than an intensity of the first UV light 504 that reaches the door 506. A central controller of the system 500 may perform a method similar to the method 400 of FIG. 4 and may determine an optimal configuration of UV light sources and corresponding durations of illumination of each (along with other parameters such as a peak wavelength) to disinfect various portions of the lavatory. For example, if the central controller determines that each feature of the lavatory was used, the central controller may control the first UV light source 502 to emit the first UV light 504 for a first period of time, may control the second UV light source 510 to emit the second UV light 512 for a second period of time, and may control the fourth UV light source 522 to emit the fourth UV light 524 for a third period of time (as shown in FIG. 5E). The central controller may control the light sources 502, 510, 522 to emit the UV light simultaneously, sequentially, or any combination thereof based on various factors such as a desired intensity of light at each surface, a duration of use of each surface (e.g., the toilet seat may receive a greater duration or intensity of illumination if the previous user spent a relatively long period of time on the toilet 514), or the like.

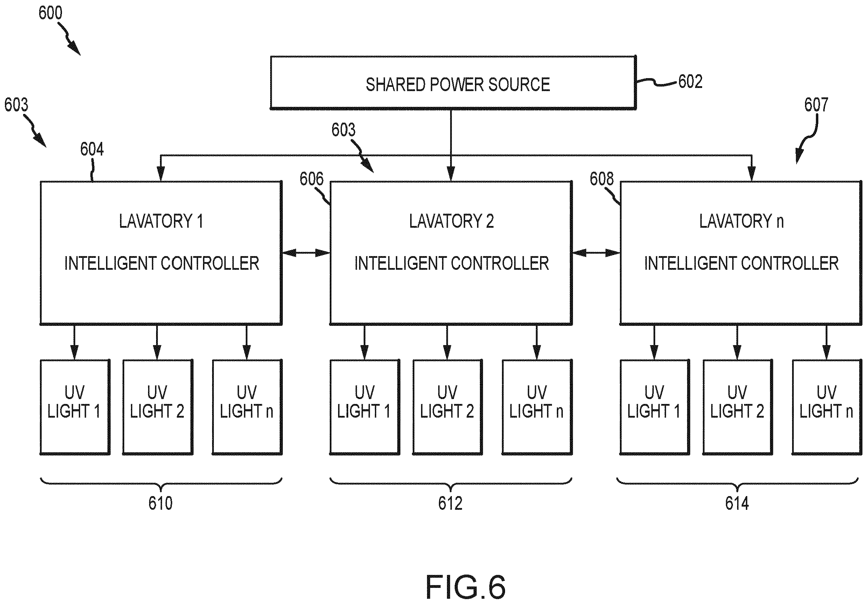

Referring now to FIG. 6, a system 600 for centrally controlled sanitization of an aircraft lavatory is shown. The system 600 may include multiple systems 603, 605, 607 that each include similar features as the system 100 of FIG. 1. In particular, each system 603, 605, 607 may include a central controller 604, 606, 608. Each system may correspond to an aircraft lavatory on a same aircraft. In that regard, the system 603 may correspond to a first aircraft lavatory, the system 605 may correspond to a second aircraft lavatory, and the system 607 may correspond to a third aircraft lavatory. Each system 603, 605, 607 may also include multiple UV light sources. In particular, the system 603 includes a first plurality of UV light sources 610, the system 605 includes a second plurality of UV light sources 612, and the system 607 includes a third plurality of UV light sources 614.

The system 600 may further include a power source 602. The power source 602 may include any power source on board the aircraft such as a battery or battery bank, a generator that is driven by a gas turbine engine or other mechanical power source and converts mechanical energy into alternating current (AC) or direct current (DC) electrical power, or the like. The system 600 or aircraft may include the single power source 602 that provides power for each system 603, 605, 607. In that regard, the central controllers 604, 606, 608 may communicate with each other regarding power draw of their respective systems. The central controllers 604, 606, 608 may also prioritize power draw by various components based on a predetermined prioritization. For example, the aircraft operator may determine that visible light takes priority over flushing a toilet, which in turn takes priority over sanitization, and that sanitization of toilets and sinks takes priority over sanitization of floors. The central controllers 604, 606, 608 may further determine or predict an upcoming sequence of events. For example, if a user of the system 604 has been in the first lavatory for longer than a user of the system 606 has been in the second lavatory, then the central controllers 604, 606, 608 may determine that the first lavatory will become vacant prior to the second lavatory. The central controllers 604, 606, 608 may predict upcoming power draw of the various components based on these predictions. The central controllers 604, 606, 608 may also be aware of a power threshold corresponding to a maximum amount of power that may be drawn from the power source 602. The central controllers 604, 606, 608 may control power distribution within their respective systems 603, 605, 607 based on the current and respective power draw of the components of the systems 603, 605, 607, based on the prioritization of components, based on a predicted sequence of events, and based on the power threshold. In particular, the central controllers 604, 606, 608 may control the various components to ensure that a total power draw by the system 600 remains at or below the power threshold and to ensure that the prioritized components receive power prior to unprioritized components.

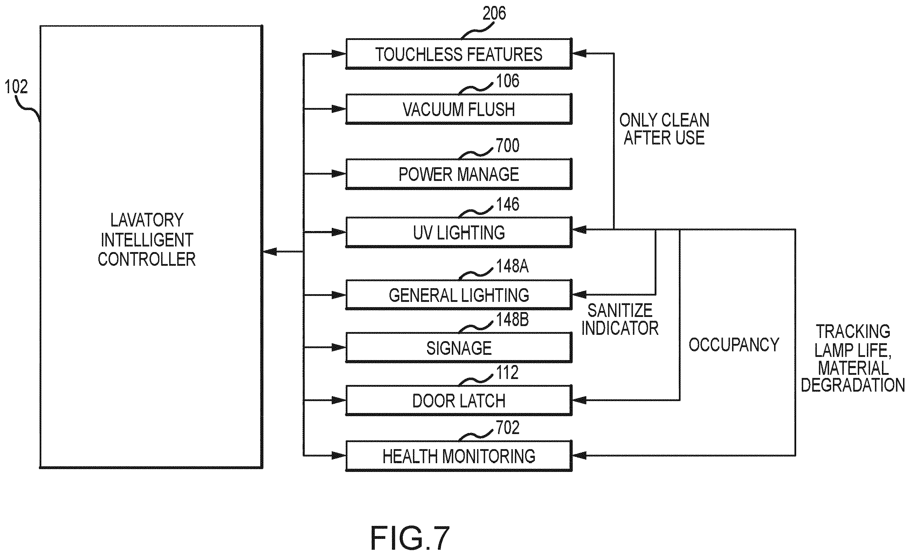

Referring now to FIGS. 4 and 7, an exemplary use of the method 400 for control of UV disinfecting lighting to disinfect portions of the lavatory is shown. The exemplary use of FIG. 7 is shown using the system 100 of FIGS. 1, 2, and 3. Only certain features of the system 100 of FIGS. 1, 2, and 3 are shown and discussed in the example. In particular, the example discusses the central controller 102 which is coupled to each of the touchless flush sensor 206, the vacuum flush 106, the sink valve 140, the UV light source 146, general visible light sources for illuminating the lavatory 148A, visible light sources for illuminating signs 148B, the door latch 112, a health monitoring function 702 (which may be implemented in the central controller 102 or in another location), and a power management function 700 (which may be implemented in the central controller 102 or in another location.

As discussed above, the UV light source 146 may include multiple UV light sources that each illuminate a specific area of the lavatory. For example, a toilet UV light source may illuminate the toilet, a door UV light source may illuminate a door handle, and a sink UV light source may illuminate the sink. Because it may be undesirable for the UV light to reach a human, it may be desirable to only emit the UV light in response to the lavatory being vacant. Additionally, it may be desirable to only illuminate portions of the lavatory that were recently used using the UV light to reduce a reduction in lifetime of the UV light sources and to reduce degradation of surfaces. In that regard, the central controller 102 may make inferences regarding which portions of the lavatory were used by the user. For example, the central controller may determine that the toilet was used in response to the touchless flush sensor 206 receiving a request to flush the toilet, may determine that the door handle was used in response to the door latch 112 being engaged, and may determine that the sink was used in response to the sink valve 140 turning on.

After the central controller 102 determines that the user has left the lavatory, the central controller 102 may control the UV light source 146 to illuminate the portions of the lavatory that were used by the user in order to disinfect those areas. In addition, the central controller 102 may control the visible light source 148B to indicate that disinfecting is occurring and to indicate that the lavatory should remain vacant.

Throughout the process, the health monitoring 500 may track a remaining life of each light source and may track a duration of exposure of each surface to the UV light. The central controller 102 may transmit a notification to an operator of the aircraft in response to the health monitoring 500 indicating that the remaining life (e.g., of a UV bulb) has reached or dropped below a threshold remaining life, or in response to the health monitoring 500 indicating that one or more surface of the lavatory has degraded to or beyond a threshold degradation level.

In addition, the power management 700 may monitor power usage by the various components as well as components of systems of other lavatories on board the aircraft. The power management 700 may further determine a power threshold and a prioritization of each component. The power management 700 may ensure that a total power draw during the exemplary use shown in FIG. 7 remains at or below the power threshold.

Benefits and other advantages have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, and any elements that may cause any benefit or advantage to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C.

Systems, methods and apparatus are provided herein. In the detailed description herein, references to "various embodiments", "one embodiment", "an embodiment", "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

## Claims

1. A system for centrally controlled sanitization of a lavatory of an aircraft using ultraviolet, UV, light, the system comprising:
a first UV light source (502) configured to emit a first UV light (504);
a second UV light source (510) configured to emit a second UV light (512); and
a central controller (102) coupled to the first UV light source and the second UV light source and configured to independently control the first UV light source and the second UV light source to emit the first UV light and the second UV light at least one of at different times or for different durations.

2. The system of claim 1, further comprising at least one sensor (130) configured to detect data corresponding to usage of the lavatory, wherein the central controller is further configured to control at least one of the first UV light source or the second UV light source based on the data corresponding to the usage of the lavatory.

3. A system for centrally controlled sanitization of a lavatory of an aircraft using ultraviolet, UV, light, the system comprising:
a first UV light source (502) configured to emit a first UV light (504);
a second UV light source (510) configured to emit a second UV light (512);
at least one sensor (130) configured to detect data corresponding to usage of the lavatory; and
a central controller (102) coupled to the first UV light source, the second UV light source, and the at least one sensor, and configured to independently control the first UV light source and the second UV light source based on the data corresponding to the usage of the lavatory.

4. The system of claim 2 or 3, wherein the data corresponding to the usage of the lavatory indicates a used portion of the lavatory, wherein the central controller is further configured to control the at least one of the first UV light source or the second UV light source to illuminate the used portion of the lavatory with the UV light.

5. The system of claim 4, wherein the at least one sensor includes at least one of:
a touchless seat sensor (204) configured to receive a toilet seat request corresponding to a request to lift a toilet seat of the lavatory;
a touchless soap sensor (210) configured to receive a soap dispense request corresponding to a request to dispense soap within the lavatory;
a touchless faucet sensor (212) configured to detect a faucet request corresponding to a request to operate a faucet of the lavatory;
a touchless flush sensor (206) configured to detect a flush request corresponding to a request to flush a toilet of the lavatory; or
a touchless trash sensor (214) configured to detect a trash flap request corresponding to a request to actuate a trash flap of the lavatory.

6. The system of any preceding claim, wherein the central controller is further configured to determine a power threshold corresponding to a maximum amount of power available to the system, and to control the first UV light source and the second UV light source to cause a total power draw of the system to remain at or below the power threshold.

7. The system of any preceding claim, further comprising a memory (126) configured to store parameters corresponding to the first UV light source and the second UV light source, wherein:
the parameters include at least one of a turn-on time, a power of the first UV light or the second UV light, or a peak wavelength of the first UV light or the second UV light; and
the central controller is further configured to control the first UV light source and the second UV light source based on the parameters.

8. The system of claim 7, wherein the parameters are adjustable by at least one of an operator of the system or the central controller.

9. The system of any preceding claim, wherein the central controller is further configured to determine a health status of the system including at least one of a remaining life of the first UV light source, a remaining life of the second UV light source, or surface wear experienced by a surface of the lavatory in response to the first UV light or the second UV light.

10. The system of any preceding claim, wherein the first UV light and the second UV light each have a peak wavelength that is between 200 nanometers and 230 nanometers (0.00787 thousandths of an inch and 0.00906 thousandths of an inch).

11. A method for centrally controlled sanitization of a lavatory of an aircraft using ultraviolet, UV, light, the method comprising:
providing, in the lavatory, a first UV light source (502) configured to emit a first UV light (504) and a second UV light source (510) configured to emit a second UV light (512); and
independently controlling, by a central controller (102), the first UV light source and the second UV light source to emit the first UV light and the second UV light at least one of at different times or for different durations.

12. The method of claim 11, further comprising detecting, by a sensor (130), data corresponding to usage of the lavatory, wherein independently controlling the first UV light source and the second UV light source includes controlling at least one of the first UV light source or the second UV light source based on the data corresponding to the usage of the lavatory.

13. The method of claim 12, wherein the data corresponding to the usage of the lavatory indicates a used portion of the lavatory, and wherein independently controlling the first UV light source and the second UV light source includes controlling the at least one of the first UV light source or the second UV light source to illuminate the used portion of the lavatory with the UV light.

14. The method of claim 11, 12 or 13, further comprising:
determining, by the central controller, a power threshold corresponding to a maximum amount of power available to the lavatory; and
causing, by the central controller, a total power draw of components of the lavatory to remain at or below the power threshold; and/or further comprising
determining, by the central controller, a health status of components of the lavatory including at least one of a remaining life of the first UV light source, a remaining life of the second UV light source, or surface wear experienced by a surface of the lavatory in response to the first UV light or the second UV light.

15. The method of any of claims 11 to 14, further comprising storing, in a memory (126), parameters corresponding to the first UV light source and the second UV light source, wherein:
the parameters include at least one of a turn-on time, a power of the first UV light or the second UV light, or a peak wavelength of the first UV light or the second UV light; and
independently controlling the first UV light source and the second UV light source is based on the parameters.
